# FASCICULE DE BREVET EUROPEEN

(11) **EP 4 554 568 B1**
(45) Date de publication et mention de la délivrance du brevet: **08.04.2026**
(21) Numéro de dépôt: 23764351.5
(22) Date de dépôt: 11.07.2023
(51) Int. Cl.: A61K 31/137, A61P 11/06, A61P 11/08, A61K 9/00, A61K 47/10, A61K 47/06, A61P 11/00, A61K 9/10

(54) **COMPOSITION PHARMACEUTIQUE COMPRENANT DU SALBUTAMOL**
PHARMAZEUTISCHE ZUSAMMENSETZUNG ENTHALTEND SALBUTAMOL
PHARMACEUTICAL COMPOSITION COMPRISING SALBUTAMOL

(30) Priorité: 13.07.2022 FR 2207237
(43) Date de publication de la demande: 21.05.2025
(73) Titulaire: Aptar France SAS, 27110 Le Neubourg (FR)
(72) Inventeur: PEYRON, Isabelle, 81100 Castres (FR); ROSSI, Irene, 63074 San Benedetto del Tronto (Ascoli Piceno) (IT)
(74) Mandataire: Santarelli
(86) Numéro de dépôt international: PCT/FR2023/051079
(87) Numéro de publication internationale: WO 2024/013455

(56) Documents cités:
- EP-A1- 2 749 275
- WO-A1-2017/093758
- CN-A- 103 655 226
- US-A1- 2014 230 812
- US-A1- 2021 244 688

## Description

### Domaine technique

La présente invention se rapporte à une composition pharmaceutique qui est susceptible d'être utilisée dans le traitement de troubles respiratoires et qui comprend un principe actif à base de salbutamol, en particulier du sulfate de salbutamol, et un gaz propulseur formé par du 1,1-difluoroéthane.

L'invention se rapporte également à une cartouche comprenant cette composition pharmaceutique ainsi qu'à un aérosol-doseur muni d'une telle cartouche.

L'invention se rapporte enfin aux utilisations de cette composition pharmaceutique et de cette cartouche dans un aérosol-doseur.

### État de la technique antérieure

Le salbutamol ainsi que ses dérivés sont des principes actifs connus comme bronchodilatateurs dans le traitement de troubles respiratoires tels que l'asthme et les broncho-pneumopathies chroniques obstructives (BPCO).

Les compositions pharmaceutiques comprenant du salbutamol ou l'un de ses dérivés sont classiquement délivrées aux patients au moyen d'un aérosol-doseur (en anglais "metered dose inhaler" ou MDI).

Un aérosol-doseur est un dispositif d'administration équipé d'une cartouche contenant la composition pharmaceutique, d'une valve doseuse permettant de distribuer une quantité contrôlée de composition pharmaceutique contenant le principe actif et d'un applicateur permettant d'exercer une pression sur la valve doseuse et muni d'un embout buccal.

La composition pharmaceutique comprend un gaz propulseur dans lequel le principe actif est dissous, suspendu ou dispersé, et éventuellement un ou plusieurs autres composés qui peuvent être notamment choisis parmi des tensioactifs, des excipients polaires et des conservateurs.

Le choix du gaz propulseur mis en œuvre dans les aérosols-doseurs à finalité pharmaceutique a évolué au cours des années.

Compte tenu de leurs effets délétères sur la couche d'ozone, les chlorofluorocarbones (CFC), longtemps utilisés, ont été abandonnés au profit d'hydrofluoroalcanes (HFA) tel que le 1,1,1,2-tétrafluoroéthane (HFA-134a ou R-134a) et le 1,1,1,2,3,3,3-heptafluoropropane (HFA-227ea ou R-227ea) qui sont des composés ne présentant pas d'effet néfaste sur la couche d'ozone ni de toxicité humaine.

Toutefois, ces hydrofluoroalcanes R-134a et R-227ea se caractérisant par un fort potentiel de réchauffement global (PRG) avec un impact non négligeable sur l'effet de serre, des compositions pharmaceutiques comprenant du salbutamol, ou l'un de ses dérivés, et un gaz propulseur alternatif ont été proposées. EP 2749275 décrit un aérosol-doseur comprenant du sulfate de salbutamol, du HFA-134a, du PEG 6000 et de l'éthanol.

Ainsi, les documents WO 2013/054137 A1, WO 2014/170689 A1 et WO 2013/054135 A1, respectivement référencés [1] à [3] dans la suite de la présente description, décrivent la mise en œuvre d'un hydrofluorocarbure (HFC) particulier en tant que gaz propulseur alternatif, le 1,1-difluoroéthane (HFC-152a ou R-152a) dans des compositions pharmaceutiques comprenant du sulfate de salbutamol.

Plus particulièrement, les compositions pharmaceutiques décrites dans les documents [1] et [2] comprennent du sulfate de salbutamol, du R-152a ainsi qu'un ou plusieurs tensioactifs dont le rôle est d'aider à la dispersion des particules de principe actif dans le gaz propulseur.

Dans le document [1], au moins l'un des tensioactifs est l'acide oléique.

De manière avantageuse, les compositions pharmaceutiques du document [1] ne comprennent que du sulfate de salbutamol, du R-152a et de l'acide oléique, en l'absence d'éthanol. Ce document [1] précise que le choix du R-152a comme gaz propulseur et de l'acide oléique comme tensioactif permet d'obtenir des compositions pharmaceutiques qui, même en l'absence d'éthanol, présentent de bonnes performances thérapeutiques lorsqu'elles sont délivrées à partir d'un dispositif d'administration de médicaments tel qu'un aérosol-doseur.

Au contraire, dans le document [2], au moins l'un des tensioactifs n'est pas l'acide oléique. Ce tensioactif peut notamment être choisi parmi l'oléate d'éthyle, la polyvinylpyrrolidone (PVP), le monooléate de sorbitane, le trioléate de sorbitane, le myristate d'isopropyle, les polyéthylène glycols tels que le polyéthylène glycol 300, le monooléate ou monolaurate de polyoxyéthylène 20 sorbitane, le polyéthylène glycol propoxylé et la lécithine, étant précisé que le tensioactif préférentiel est la PVP.

De manière avantageuse, les compositions pharmaceutiques décrites dans le document [2] comprennent du sulfate de salbutamol, du R-152a et un ou plusieurs tensioactifs mais sont exemptes d'acide oléique. De manière préférentielle, ces compositions pharmaceutiques sont également exemptes d'éthanol tout en conservant de bonnes performances thérapeutiques lorsqu'elles sont délivrées au moyen d'un dispositif du type aérosol-doseur (MDI).

Contrairement aux compositions pharmaceutiques décrites dans les documents [1] et [2], les compositions pharmaceutiques décrites dans le document [3] ne comprennent aucun tensioactif au motif que les tensioactifs ne seraient pas souhaitables et qu'il y aurait un intérêt à former une suspension stable sans recourir à leur mise en œuvre. Le document [3] précise que l'utilisation du gaz propulseur R-152a permet de préparer des compositions pharmaceutiques qui sont non seulement exemptes de tensioactifs mais également exemptes d'excipients polaires et qui présentent de bonnes performances thérapeutiques lorsqu'elles sont délivrées à partir d'un dispositif du type aérosol-doseur (MDI).

Or, l'expérience montre que les performances d'aérosolisation de compositions pharmaceutiques décrites dans ces documents [1] à [3], et plus particulièrement dans les documents [1] et [3], ne sont pas satisfaisantes.

On observe en particulier que des compositions pharmaceutiques constituées de sulfate de salbutamol, de R-152a et d'acide oléique conformes à l'enseignement du document [1] se caractérisent par des performances d'aérosolisation qui sont bien inférieures à celles de compositions pharmaceutiques conformes à l'enseignement du document [3] qui sont pourtant exemptes de tensioactifs, alors que le rôle de ces tensioactifs est d'aider à la dispersion des particules de principe actif (sulfate de salbutamol) et de la stabiliser dans le gaz propulseur (R-152a).

De surcroît, les performances d'aérosolisation de ces compositions pharmaceutiques décrites dans les documents [1] à [3] diminuent avec le temps, ce qui est préjudiciable à leur efficacité thérapeutique.

C'est donc sur la base de ces constats et dans un souci constant d'amélioration des propriétés d'aérosolisation et, partant, des propriétés thérapeutiques conférées par les compositions pharmaceutiques destinées au traitement de troubles respiratoires qu'est basée la présente invention.

### Exposé de l'invention

Ces buts ainsi que d'autres sont atteints, en premier lieu, par une composition pharmaceutique du type précité, c'est-à-dire qui comprend un principe actif à base de salbutamol (a) et du 1,1-difluoroéthane (b).

Selon l'invention, la composition pharmaceutique est constituée des composés suivants :
(a) un principe actif à base de salbutamol,
(b) du 1,1-difluoroéthane (R-152a),
(c) un polyéthylène glycol, et
(d) de l'éthanol.

Les Inventeurs ont constaté que, de manière inattendue et surprenante, une composition pharmaceutique qui est constituée d'un principe actif à base de salbutamol (a), de 1,1-difluoroéthane (b), d'un polyéthylène glycol (c) et d'-éthanol (d), permet d'atteindre des performances d'aérosolisation bien supérieures aux compositions pharmaceutiques des documents [1] à [3] qui sont toutes à base de salbutamol et de 1,1-difluoroéthane mais préférentiellement exemptes d'éthanol et ce, que ces compositions pharmaceutiques comprennent un tensioactif tel que l'acide oléique comme dans le document [1] ou bien qu'elles en soient exemptes comme dans le document [3].

Comme illustré dans les exemples ci-après, la composition pharmaceutique selon l'invention se caractérise, en outre, par des performances d'aérosolisation élevées qui sont stables dans le temps et, par conséquent, par une efficacité thérapeutique performante qui s'inscrit dans la durée.

La composition pharmaceutique selon l'invention comprend un principe actif (a) à base de salbutamol.

Dans une variante avantageuse de la composition pharmaceutique selon l'invention, ce principe actif (a) est un sel de salbutamol pharmaceutiquement acceptable.

Dans une variante préférée de l'invention, ce principe actif (a) est du sulfate de salbutamol.

Le principe actif (a) se présente avantageusement sous forme de particules dont la taille est adaptée à une délivrance par inhalation de la composition pharmaceutique dans laquelle il est contenu. De manière classique, le diamètre médian des particules de principe actif (a) est inférieur ou égal à 5 µm et, de préférence, compris entre 0,5 µm et 4 µm.

Dans une variante de la composition selon l'invention, la proportion massique de principe actif (a) est comprise entre 0,05 % et 0,5 % en masse par rapport à la masse totale de la composition pharmaceutique. Cette proportion massique est avantageusement comprise entre 0,1 % et 0,4 % en masse et, de préférence, comprise entre 0,2 % et 0,35 % en masse par rapport à la masse totale de la composition pharmaceutique.

La composition pharmaceutique selon l'invention comprend également du 1,1-difluoroéthane (b) en tant que gaz propulseur.

Dans une variante de la composition selon l'invention, la proportion massique de 1,1-difluoroéthane (b) est comprise entre 87,5 % et 99,89 % en masse par rapport à la masse totale de la composition pharmaceutique.

Dans une autre variante, la proportion massique de 1,1-difluoroéthane (b) est comprise entre 92,5 % et 99,89 % en masse par rapport à la masse totale de la composition pharmaceutique. Cette proportion massique est avantageusement comprise entre 95,6 % et 99,68 % en masse et, de préférence, comprise entre 97,15 % et 99,26 % en masse par rapport à la masse totale de la composition pharmaceutique.

La composition pharmaceutique selon l'invention comprend, en outre, du polyéthylène glycol (c) en tant que tensioactif.

Le polyéthylène glycol (c) ou PEG est un polyéther linéaire synthétisé à partir de monomères d'oxyde d'éthylène et répondant à la formule H-(CH₂-CH₂-O)ₙ-OH, n étant un nombre entier tel que n ≥ 4.

Dans une variante de la composition selon l'invention, le polyéthylène glycol (c) présente une masse molaire moyenne en masse M_{w} inférieure ou égale à 20000 g/mol. Cette masse molaire moyenne en masse M_{w} du PEG est avantageusement comprise entre 100 g/mol et 5000 g/mol et, de préférence, comprise entre 200 g/mol et 2000 g/mol.

En particulier, des PEG présentant une masse molaire moyenne en masse M_{w} de 400g/mol, 600 g/mol et 1000 g/mol, respectivement désignés sous les acronymes PEG 400, PEG 600 et PEG 1000, conviennent tout particulièrement pour la composition pharmaceutique selon l'invention,

Dans une variante de la composition selon l'invention, la proportion massique de polyéthylène glycol (c) est comprise entre 0,01 % et 2 % en masse par rapport à la masse totale de la composition pharmaceutique. Cette proportion massique totale est avantageusement comprise entre 0,02 % et 1 % en masse et, de préférence, comprise entre 0,04 % et 0,5 % en masse par rapport à la masse totale de la composition pharmaceutique.

La composition pharmaceutique selon l'invention comprend enfin de l'éthanol (d) en tant qu'excipient polaire.

Dans une variante de la composition selon l'invention, la proportion massique d'éthanol (d) est comprise entre 0,05 % et 10 % en masse par rapport à la masse totale de la composition pharmaceutique.

Dans une autre variante, la proportion massique d'éthanol (d) est comprise entre 0,05 % et 5 % en masse par rapport à la masse totale de la composition pharmaceutique.

Les performances d'aérosolisation de la composition pharmaceutique selon l'invention peuvent être atteintes avec une proportion massique d'éthanol relativement faible, qui ne présente aucun danger pour la santé du patient, même jeune.

La proportion massique d'éthanol (d) peut avantageusement être comprise entre 0,2 % et 3 % en masse et, de préférence, comprise entre 0,5 % et 2 % en masse par rapport à la masse totale de la composition pharmaceutique.

La présente invention se rapporte, en deuxième lieu, à une composition pharmaceutique pour une utilisation dans le traitement de patients souffrant ou susceptibles de souffrir de troubles respiratoires,

Selon l'invention, cette composition pharmaceutique, qui est utilisée dans le traitement de troubles respiratoires, est telle que définie ci-dessus, c'est-à-dire qu'elle est constituée des composés suivants :
(a) un principe actif à base de salbutamol,
(b) du 1,1-difluoroéthane (R-152a),
(c) un polyéthylène glycol, et
(d) de l'éthanol.

Les caractéristiques décrites précédemment en liaison avec la composition pharmaceutique et, notamment, les caractéristiques relatives aux différents composés (a), (b), (c) et (d) constituant cette composition pharmaceutique ainsi qu'à leurs proportions massiques respectives, sont bien entendu applicables à la présente utilisation dans le traitement de troubles respiratoires.

De tels troubles respiratoires peuvent être l'asthme ou encore les broncho-pneumopathies chroniques obstructives (BPCO).

Dans le cadre de la présente invention, les patients peuvent être traités par l'administration d'une quantité efficace d'un point de vue thérapeutique d'une composition pharmaceutique telle que définie ci-dessus.

La présente invention se rapporte, en troisième lieu, à une cartouche comprenant une composition pharmaceutique ainsi qu'à un aérosol-doseur muni d'une telle cartouche.

Selon l'invention, cette composition pharmaceutique est telle que définie ci-dessus, c'est-à-dire qu'elle est constituée des composés suivants :
(a) un principe actif à base de salbutamol,
(b) du 1,1-difluoroéthane (R-152a),
(c) un polyéthylène glycol, et
(d) de l'éthanol.

Comme précédemment, les caractéristiques relatives à chacun de ces composés (a) à (d) peuvent être prises seules ou en combinaison.

La présente invention se rapporte, en quatrième lieu, à l'utilisation d'une composition pharmaceutique et/ou d'une cartouche telles que définies ci-dessus dans un aérosol-doseur (MDI), un tel dispositif étant classiquement utilisé pour délivrer des compositions pharmaceutiques comprenant un principe actif à base de salbutamol ou d'un sel pharmaceutiquement acceptable de celui-ci.

D'autres caractéristiques et avantages de l'invention apparaîtront mieux à la lecture du complément de description qui suit, qui se rapporte à des exemples de compositions pharmaceutiques ainsi qu'à l'évaluation de leurs performances d'aérosolisation *in vitro,* deux des compositions pharmaceutiques étant des compositions pharmaceutiques conformes à l'invention, notées C et C', les trois autres étant des compositions pharmaceutiques comparatives conformes aux enseignements des documents [1] et [3], notées C_{[1]}, C_{[1]}' et C_{[3]},

### Brève description des dessins

La figure 1 représente les graphes illustrant la fraction déposée de particules de salbutamol (exprimée en %) provenant de cinq doses de compositions pharmaceutiques C, C', C_{[1]}, C_{[1]}' et C_{[3]} telles qu'obtenues à T0, en fonction des étages de l'impacteur pharmaceutique NGI, les compositions pharmaceutiques C et C' étant conformes à l'invention et les trois autres étant des compositions pharmaceutiques comparatives, les compositions C_{[1]} et C_{[1]}' étant conformes à l'enseignement du document [1] et la composition C_{[3]} étant conforme à l'enseignement du document [3].
La figure 2 représente les graphes illustrant la fraction déposée de particules de salbutamol (exprimée en %) provenant de cinq doses des compositions pharmaceutiques C, C', C_{[1]}, C_{[1]}' et C_{[3]} telles qu'obtenues à T3M, en fonction des étages de l'impacteur pharmaceutique NGI.
La figure 3 représente les graphes illustrant la fraction déposée de particules de salbutamol (exprimée en %) provenant de cinq doses de la composition pharmaceutique C conforme à l'invention à T0 et à T3M, en fonction des étages de l'impacteur pharmaceutique NGI.
La figure 4 représente les graphes illustrant la fraction déposée de particules de salbutamol (exprimée en %) provenant de cinq doses de la composition pharmaceutique C' conforme à l'invention à T0 et à T3M, en fonction des étages de l'impacteur pharmaceutique NGI.

### Exposé détaillé de modes de réalisation particuliers

Cinq compositions pharmaceutiques C, C', C_{[1]}, C_{[1]}' et C_{[3]} ont été préparées à partir des composés suivants :
- du sulfate de salbutamol en tant que principe actif (a),
- du R-152a en tant que gaz propulseur (b),
- du polyéthylène glycol (c) ayant une masse moléculaire de 400 g/mol (PEG 400) ou de l'acide oléique en tant que tensioactif, et/ou
- de l'éthanol (d) en tant qu'excipient polaire.

Les aérosols-doseurs qui ont fait l'objet des tests ont été préparés avec les mêmes lots de composés, de cartouches en aluminium et de valves doseuses, selon un protocole opératoire identique.

Dans une première étape, une valve doseuse a été sertie sur chacune des cartouches avec un équipement adéquat,

Dans une deuxième étape, on a procédé au remplissage, en deux temps, de cinq séries distinctes d'aérosols-doseurs par introduction via la valve doseuse :
- d'une susperision concentrée comprenant 30,125 mg de sulfate de salbutamol, une quantité réduite de gaz propulseur R-152a et, le cas échéant, la ou les proportions massiques de tensioactif (PEG 400 ou acide oléique) et d'éthanol telles qu'indiquées dans le tableau 1 ci-dessous, par rapport à la masse totale de composition pharmaceutique, puis
- d'une quantité suffisante de gaz propulseur R-152a pour atteindre une masse totale de composition pharmaceutique de 9,57 g.

**Tableau 1**

| Composition | C | C' | C_{[1]} | C_{[1]}' | C_{[3]} |
|---|---|---|---|---|---|
| PEG 400 (% en masse) | 0,05 | 0,1 | 0 | 0 | 0 |
| Acide oléique (% en masse) | 0 | 0 | 0,03 | 0,05 | 0 |
| Ethanol (% en masse) | 1,00 | 1,00 | 0 | 0 | 0 |

Deux séries de tests de mesure de distribution granulométrique aérodynamique (en anglais "aerodynamic particle size distribution" ou APSD) ont été réalisées.

Ces deux séries de tests, qui consistent à évaluer la taille aérodynamique des particules de principe actif sortant de la valve, ont été réalisées au moyen d'un impacteur pharmaceutique à multi-étages permettant une modélisation approximative de l'arbre bronchique. Dans le cas présent, l'impacteur pharmaceutique utilisé a été le Next Generation Impactor (NGI) qui correspond à l'appareil E de la Pharmacopée Européenne.

Plus particulièrement, ces deux séries de tests ont été réalisées à un débit de 30 L/min, en expulsant 5 doses de chacune des compositions pharmaceutiques dans l'impacteur NGI.

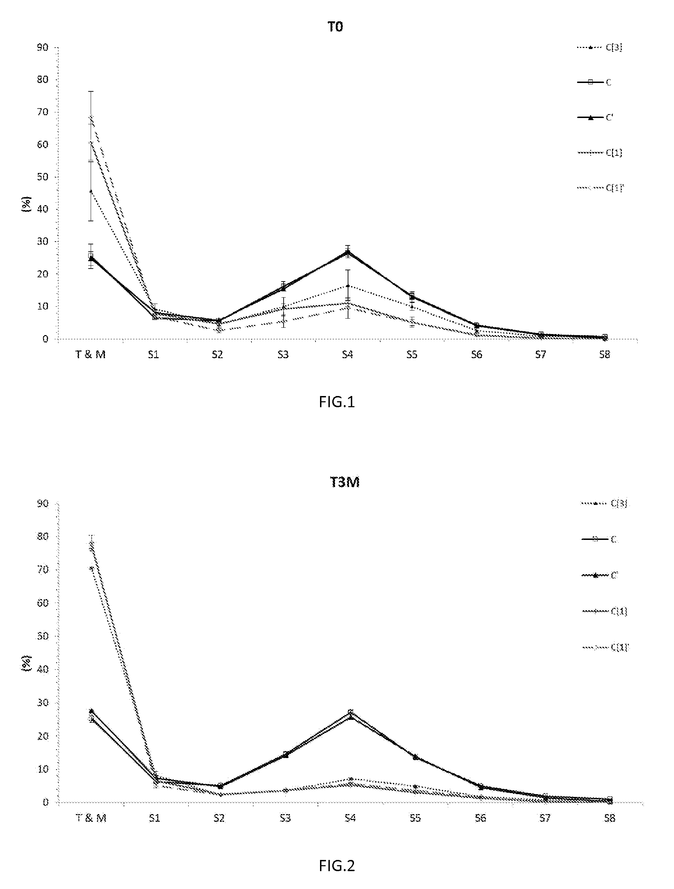

Une première série de tests de mesure de distribution granulométrique aérodynamique a été conduite sur les compositions pharmaceutiques C, C', C_{[1]}, C_{[1]}' et C_{[3]} telles qu'obtenues à T0, c'est-à-dire au terme de l'étape de remplissage en deux temps décrite ci-dessus. Les résultats de cette première série de tests sont reportés sur la figure 1.

Une seconde série de tests de mesure de distribution granulométrique aérodynamique a été conduite sur ces mêmes compositions pharmaceutiques C, C', C_{[1]}, C_{[1]}' et C_{[3]} telles qu'obtenues à T3M, c'est-à-dire au terme d'un stockage d'une durée de trois mois des aérosols-doseurs comprenant lesdites compositions C, C', C_{[1]}, C_{[1]}' et C_{[3]}, ces aérosols-doseurs étant disposés en position inversée (valve vers le bas) pendant ces trois mois. Les résultats de cette seconde série de tests sont reportés sur la figure 2.

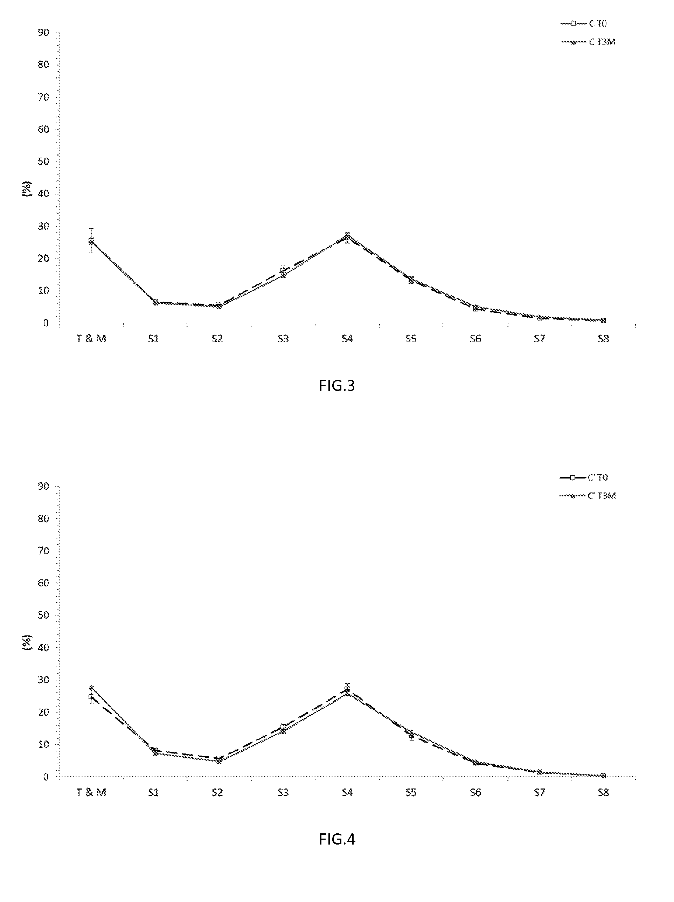

En complément, les résultats des première et seconde séries de tests conduits sur les compositions pharmaceutiques C et C' conformes à l'invention sont reportés sur la figure 3 pour la composition C et sur la figure 4 pour la composition C'.

Les graphes des figures 1 à 4 représentent les fractions de salbutamol (a) déposées, d'une part, au niveau de la gorge et de la bouche (notée T&M) et, d'autre part, sur chacun des huit étages de l'impacteur (notés S1 à S8).

Pour assurer une efficacité thérapeutique optimale, la fraction déposée au niveau de la gorge et de la bouche T&M doit être minimisée tandis que les fractions déposées sur les étages S3 à S5 doivent au contraire être maximisées.

La figure 1 montre que les compositions pharmaceutiques C et C' selon l'invention permettent effectivement d'optimiser cette efficacité thérapeutique.

D'une part, on observe que la fraction de salbutamol déposée à partir des compositions C et C' au niveau T&M est de l'ordre de 25 %. Cette fraction est très nettement inférieure aux fractions de salbutamol qui sont déposées à ce même niveau T&M à partir des compositions pharmaceutiques comparatives C_{[1]}, C_{[1]}' et C_{[3]}, fractions qui sont respectivement de l'ordre de 68 %, 60 % et 46 %.

D'autre part, on constate que la somme des fractions de salbutamol déposées sur les étages S3 à S5 à partir des compositions pharmaceutiques C et C' selon l'invention est très nettement supérieure à la somme des fractions de salbutamol déposées sur ces mêmes étages S3 à S5 à partir des compositions pharmaceutiques comparatives C_{[1]}, C_{[1]}' et C_{[3]}. Ce constat est encore plus marqué avec les compositions comparatives C_{[1]} et C_{[1]}' qui mettent pourtant en œuvre un tensioactif, en l'espèce l'acide oléique, dont le rôle est d'aider à la dispersion des particules de principe actif et de les stabiliser dans le gaz propulseur.

On observe, en outre, que les compositions pharmaceutiques C et C', qui comportent respectivement une proportion massique de 0,05 % et 0, 1 % de PEG 400, se caractérisent par des performances en termes d'efficacité thérapeutique qui sont tout à fait comparables, dans la mesure où leurs graphes respectifs sur la figure 1 se superposent au niveau T&M et sur les huit étages S1 à S8 de l'impacteur. Ainsi, une quantité même réduite de PEG dans la composition pharmaceutique selon l'invention permet d'atteindre une excellente efficacité thérapeutique.

La figure 2 montre que les compositions pharmaceutiques C et C' selon l'invention conservent cette efficacité thérapeutique optimisée après trois mois de stockage, Au contraire, on observe que cette efficacité thérapeutique est fortement dégradée pour l'ensemble des compositions pharmaceutiques comparatives C_{[1]}, C_{[1]}' et C_{[3]}.

Les figures 3 et 4 confirment que les compositions pharmaceutiques C et C' selon l'invention conservent effectivement bien cette même excellente efficacité thérapeutique après trois mois de stockage. En effet, les graphes de ces figures 3 et 4 sont pratiquement superposables, traduisant le fait que la fraction de salbutamol déposée à partir des compositions C et C' au niveau T&M est similaire ou identique à T0 et à T3M, tout comme l'est la somme des fractions de salbutamol déposées sur les étages S3 à S5 à partir de ces mêmes compositions pharmaceutiques C et C' à T0 et T3M,

### Bibliographie

[1] WO 2013/054137 A1
[2] WO 2014/170689 A1
[3] WO 2013/054135 A1

## Revendications

1. Composition pharmaceutique constituée des composés suivants :
(a) un principe actif à base de salbutamol,
(b) du 1,1-difluoroéthane (R-152a),
(c) un polyéthylène glycol, et
(d) de l'éthanol.

2. Composition pharmaceutique selon la revendication 1, dans laquelle le principe actif (a) est un sel de salbutamol pharmaceutiquement acceptable.

3. Composition pharmaceutique selon la revendication 2, dans laquelle le principe actif (a) est du sulfate de salbutamol.

4. Composition pharmaceutique selon l'une quelconque des revendications 1 à 3, dans laquelle le polyéthylène glycol (c) présente une masse molaire moyenne en masse M_{w} inférieure ou égale à 20000 g/mol, avantageusement comprise entre 100 g/mol et 5000 g/mol et, de préférence, comprise entre 200 g/mol et 2000 g/mol.

5. Composition pharmaceutique selon l'une quelconque des revendications 1 à 4, dans laquelle la proportion massique de principe actif (a) est comprise entre 0,05 % et 0,5 % en masse, avantageusement comprise entre 0,1 % et 0,4 % en masse et, de préférence, comprise entre 0,2 % et 0,35 % en masse par rapport à la masse totale de la composition pharmaceutique.

6. Composition pharmaceutique selon l'une quelconque des revendications 1 à 5, dans laquelle la proportion massique de 1,1-difluoroéthane (b) est comprise entre 87,5 % et 99,89 % en masse, notamment comprise entre 92,5 % et 99,89 % en masse, avantageusement comprise entre 95,6 % et 99,68 % en masse et, de préférence, comprise entre 97,15 % et 99,26 % en masse par rapport à la masse totale de la composition pharmaceutique.

7. Composition pharmaceutique selon l'une quelconque des revendications 1 à 6, dans laquelle la proportion massique de polyéthylène glycol (c) est comprise entre 0,01 % et 2 % en masse, avantageusement comprise entre 0,02 % et 1 % en masse et, de préférence, comprise entre 0,04 % et 0,5 % en masse par rapport à la masse totale de la composition pharmaceutique.

8. Composition pharmaceutique selon l'une quelconque des revendications 1 à 7, dans laquelle la proportion massique d'éthanol (d) est comprise entre 0,05 % et 10 % en masse, notamment comprise entre 0,05 % et 5 % en masse, avantageusement comprise entre 0,2 % et 3 % en masse et, de préférence, comprise entre 0,5 % et 2 % en masse par rapport à la masse totale de la composition pharmaceutique.

9. Composition pharmaceutique selon l'une quelconque des revendications 1 à 8 pour une utilisation dans le traitement de troubles respiratoires, tels que l'asthme et les broncho-pneumopathies chroniques obstructives (BPCO).

10. Cartouche comprenant une composition pharmaceutique selon l'une quelconque des revendications 1 à 8.

11. Aérosol-doseur muni d'une cartouche selon la revendication 10.

12. Utilisation de la composition pharmaceutique selon l'une quelconque des revendications 1 à 8 ou de la cartouche selon la revendication 10 dans un aérosol-doseur.

## Patentansprüche

1. Pharmazeutische Zusammensetzung, bestehend aus folgenden Verbindungen:
(a) einem Salbutamol-basierten Wirkstoff,
(b) 1,1-Difluorethan (R-152a),
(c) Polyethylenglykol, und
(d) Ethanol.

2. Pharmazeutische Zusammensetzung nach Anspruch 1, wobei der Wirkstoff (a) ein pharmazeutisch annehmbares Salbutamolsalz ist.

3. Pharmazeutische Zusammensetzung nach Anspruch 2, wobei der Wirkstoff (a) Salbutamolsulfat ist.

4. Pharmazeutische Zusammensetzung nach einem der Ansprüche 1 bis 3, wobei das Polyethylenglykol (c) eine massendurchschnittliche Molmasse M_{w} kleiner oder gleich 20000 g/mol aufweist, die vorteilhafterweise zwischen 100 g/mol und 5000 g/mol und vorzugsweise zwischen 200 g/mol und 2000 g/mol liegt.

5. Pharmazeutische Zusammensetzung nach einem der Ansprüche 1 bis 4, wobei der Massenanteil des Wirkstoffs (a) zwischen 0,05 und 0,5 Massen-%, vorteilhafterweise zwischen 0,1 und 0,4 Massen-% und vorzugsweise zwischen 0,2 und 0,35 Massen-%, bezogen auf die Gesamtmasse der pharmazeutischen Zusammensetzung, liegt.

6. Pharmazeutische Zusammensetzung nach einem der Ansprüche 1 bis 5, wobei der Massenanteil von 1,1-Difluorethan (b) zwischen 87,5 und 99,89 Massen-%, insbesondere zwischen 92,5 und 99,89 Massen-%, vorteilhafterweise zwischen 95,6 und 99,68 Massen-% und vorzugsweise zwischen 97,15 und 99,26 Massen-%, bezogen auf die Gesamtmasse der pharmazeutischen Zusammensetzung, liegt.

7. Pharmazeutische Zusammensetzung nach einem der Ansprüche 1 bis 6, wobei der Massenanteil von Polyethylenglykol (c) zwischen 0,01 und 2 Massen-%, vorteilhafterweise zwischen 0,02 und 1 Massen-% und vorzugsweise zwischen 0,04 und 0,5 Massen-%, bezogen auf die Gesamtmasse der pharmazeutischen Zusammensetzung, liegt.

8. Pharmazeutische Zusammensetzung nach einem der Ansprüche 1 bis 7, wobei der Massenanteil von Ethanol (d) zwischen 0,05 und 10 Massen-%, insbesondere zwischen 0,05 und 5 Massen-%, vorteilhafterweise zwischen 0,2 und 3 Massen-% und vorzugsweise zwischen 0,5 und 2 Massen-%, bezogen auf die Gesamtmasse der pharmazeutischen Zusammensetzung, liegt.

9. Pharmazeutische Zusammensetzung nach einem der Ansprüche 1 bis 8 zur Verwendung bei der Behandlung von Atemwegserkrankungen wie Asthma und chronisch obstruktiven Lungenerkrankungen (COPD).

10. Kartusche, umfassend eine pharmazeutische Zusammensetzung nach einem der Ansprüche 1 bis 8.

11. Aerosol-Dosierer, versehen mit einer Kartusche nach Anspruch 10.

12. Verwendung der pharmazeutischen Zusammensetzung nach einem der Ansprüche 1 bis 8 oder der Kartusche nach Anspruch 10 in einem Aerosol-Dosierer.

## Claims

1. Pharmaceutical composition consisting of the following compounds:
(a) an active ingredient based on salbutamol,
(b) 1,1-difluoroethane (R-152a),
(c) a polyethylene glycol, and
(d) ethanol.

2. Pharmaceutical composition according to claim 1, wherein the active ingredient (a) is a pharmaceutically acceptable salt of salbutamol.

3. Pharmaceutical composition according to claim 2, wherein the active ingredient (a) is salbutamol sulphate.

4. Pharmaceutical composition according to any one of claims 1 to 3, wherein the polyethylene glycol (c) has a mass average molar mass M_{w} less than or equal to 20000g/mol, advantageously between 100g/mol and 5000g/mol and, preferably, between 200g/mol and 2000g/mol.

5. Pharmaceutical composition according to any one of claims 1 to 4, wherein the mass proportion of active ingredient (a) is between 0.05% and 0.5% by mass, advantageously between 0.1% and 0.4% by mass and, preferably, between 0.2% and 0.35% by mass relative to the total mass of the pharmaceutical composition.

6. Pharmaceutical composition according to any one of claims 1 to 5, wherein the mass proportion of 1,1-difluoroethane (b) is between 87.5% and 99.89% by mass, in particular between 92.5% and 99.89% by mass, advantageously between 95.6% and 99.68% by mass and, preferably, between 97.15% and 99.26% by mass relative to the total mass of the pharmaceutical composition.

7. Pharmaceutical composition according to any one of claims 1 to 6, wherein the mass proportion of polyethylene glycol (c) is between 0.01% and 2% by mass, advantageously between 0.02% and 1% by mass and, preferably, between 0.04% and 0.5% by mass relative to the total mass of the pharmaceutical composition.

8. Pharmaceutical composition according to any one of claims 1 to 7, wherein the mass proportion of ethanol (d) is between 0.05% and 10% by mass, in particular between 0.05% and 5% by mass, advantageously between 0.2% and 3% by mass and, preferably, between 0.5% and 2% by mass relative to the total mass of the pharmaceutical composition.

9. Pharmaceutical composition according to any one of claims 1 to 8 for use in the treatment of respiratory disorders, such as asthma and chronic obstructive pulmonary diseases (COPD).

10. Canister comprising a pharmaceutical composition according to any one of claims 1 to 8.

11. Metered-dose inhaler provided with a canister according to claim 10.

12. Use of the pharmaceutical composition according to any one of claims 1 to 8 or of the canister according to claim 10 in a metered-dose inhaler.
